# EUROPEAN PATENT APPLICATION

(11) **EP 4 756 037 A1**
(43) Date of publication of application: **10.06.2026**
(21) Application number: 24859444.2
(22) Date of filing: 13.08.2024
(51) Int. Cl.: C12Q 1/6848, C12N 15/09, C12N 15/10

(54) **NUCLEIC ACID PURIFICATION METHOD AND KIT FOR NUCLEIC ACID PURIFICATION**

(30) Priority: 01.09.2023 JP 2023142214
(71) Applicant: Eiken Kagaku Kabushiki Kaisha, Tokyo 101-0062 (JP)
(72) Inventor: NAKAGAWA SHIMIZU, Yuki, Shimotsuga-gun, Tochigi 329-0114 (JP); SATO, Kazuhiko, Shimotsuga-gun, Tochigi 329-0114 (JP)
(74) Representative: Boult Wade Tennant LLP
(86) International application number: PCT/JP2024/028868
(87) International publication number: WO 2025/047408

(57) **Abstract**

An object of the present invention is to provide a method of purifying a nucleic acid from a sample containing a nucleic acid easily, rapidly, and with high purity, and a kit for such purification. The nucleic acid purification method of the present invention is a method including a step of bringing a sample containing a nucleic acid into contact with a zeolite and a proton acceptor.

## Description

### Technical Field

The present invention relates to a nucleic acid purification method and a kit for nucleic acid purification.

### Background Art

Currently, nucleic acid amplification techniques typified as a PCR method and an LAMP method have become widespread in all fields of biology, including molecular biology and medicine, and are widely used in, for example, genetic diagnosis, DNA testing, food inspection, environmental hygiene inspection, and inspections of plants and animals. When nucleic acids in a specimen are amplified, it is generally necessary to extract and purify nucleic acids from the specimen. Examples of methods of extracting and purifying nucleic acids from a specimen include methods using currently commercially available nucleic acid extraction kits. On the other hand, when a general commercially available nucleic acid extraction kit (for example, QIAamp Viral RNA Mini Kit (commercially available from Qiagen)) is used, high-purity nucleic acids can be obtained. However, it requires a plurality of devices such as a high-speed centrifuge and a heating heater, and it is difficult to use the kit unless the operation environment is fully equipped with such devices. In addition, the procedure is complicated because it involves several tens of operation steps, and it takes about 1 hour to extract and purify nucleic acids (for example, Non Patent Literature 1 and 2). In nucleic acid amplification techniques, when there are a large number of test samples, it is desirable to prepare samples that can be applied for nucleic acid amplification in a short time. Therefore, it is difficult to apply extraction and purification methods that require complicated operations. In addition, when more simplified commercially available nucleic acid extraction kits (for example, Kaneka Easy DNA extraction kit (commercially available from Kaneka Corporation), Template Prepper for DNA (commercially available from Nippon Gene Co., Ltd.), and ISOSPIN Viral RNA (commercially available from Nippon Gene Co., Ltd.)) are used, nucleic acids can be extracted and purified in a short time, but the purity of the nucleic acids may be low. In addition, even when such kits are used, a heating operation may be required, and the procedure may involve several tens of operation steps, and thus it is hard to say that nucleic acids can be extracted and purified easily, rapidly, and with high purity. Accordingly, there is a market demand for a method of extracting and purifying nucleic acids easily, rapidly, and with high purity without requiring a large device.

In addition, methods of isolating and purifying nucleic acids from cell lysates and the like using zeolite are also known. For example, Patent Literature 1 discloses a method of preparing a sample for nucleic acid amplification to be used for amplifying nucleic acids contained in a biological sample, including an extraction step of adding a nucleic acid extraction reagent containing an anionic surfactant and/or an alkali to a biological sample to obtain a nucleic acid extraction solution, and a step of bringing the nucleic acid extraction solution into contact with zeolite, wherein the zeolite is a proton-type zeolite, and substances adsorbed to the zeolite are removed. Patent Literature 2 discloses, in a method of isolating nucleic acids from a biological solution (for example, cell lysate) cell lysate, a method of pretreating a clarified lysate using zeolite, and describes that, when zeolite is added to a cell lysate treated with an alkaline reagent such as sodium dodecyl sulfate (SDS), SDS and the like are adsorbed, and the cell lysate can be clarified by centrifugation.

### Citation List

### Patent Literature

[Patent Literature 1] Japanese Patent No. 5290987
[Patent Literature 2] Japanese Unexamined Patent Publication No. 2005-531329

### Non Patent Literature

[Non Patent Literature 1] National Institute of Infectious Diseases, "Manual for the Detection of Pathogens 2019-nCoV Ver. 2. 5," February 15, 2020, https://www.niid.gojp/niid/images/lab-manual/2019-nCoV20200215.p df
[Non Patent Literature 2] "QIAamp (registered trademark) Viral RNA Mini Protocol and Troubleshooting," (commercially available from QIAGEN), April 2010, https://www.qiagen.com/jp/resources/download.aspx?id=7d6918b1-77 dd-4dac-b694-a9ca8aa58b43&lang=ja-JP

### Summary of Invention

### Technical Problem

An object of the present invention is to provide a method of purifying nucleic acids from a sample containing nucleic acids easily, rapidly, and with high purity, and a kit for such purification.

### Solution to Problem

The inventors found that, surprisingly, when a sample containing nucleic acids is mixed with a zeolite and a proton acceptor such as tris(hydroxymethyl)aminomethane, the effect of the zeolite removing (adsorbing) substances other than nucleic acids such as a nucleic acid amplification reaction inhibiting substance in the sample is improved, and completed the present invention.

Specifically, the present invention relates to, for example, the following inventions.
[1] A nucleic acid purification method, including a step of bringing a sample containing a nucleic acid into contact with a zeolite and a proton acceptor.
[2] The method according to [1], wherein the proton acceptor is a compound represented by General Formula (1): 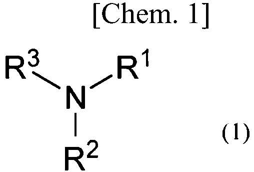 (in the formula, R¹, R² and R³ are each independently a hydrogen atom, an optionally substituted aliphatic hydrocarbon group having 1 to 6 carbon atoms, or an optionally substituted chain aliphatic hydrocarbon group having 2 to 10 carbon atoms and in which a nitrogen atom, a sulfur atom or an oxygen atom is interposed between carbon atoms, the substituent is selected from the group consisting of a hydroxyl group, a carboxyl group, an amino group, a sulfonic acid group, an imino group, a guanidyl group, an aromatic hydrocarbon group having 6 to 12 carbon atoms, and a 3- to 6-membered heterocyclic group which contains, as a ring-constituting atom, at least one atom selected from the group consisting of a nitrogen atom, a sulfur atom and an oxygen atom, and which may have a substituent that is an aliphatic hydrocarbon group having 1 to 6 carbon atoms,
   when the optionally substituted aliphatic hydrocarbon group having 1 to 6 carbon atoms has a substituent that is an aromatic hydrocarbon group having 6 to 12 carbon atoms, the aliphatic hydrocarbon group having 1 to 6 carbon atoms is an aliphatic hydrocarbon group having 2 to 6 carbon atoms, and the aromatic hydrocarbon group is not bonded to the carbon atom of the aliphatic hydrocarbon group having 2 to 6 carbon atoms that is closest to the N atom in General Formula (1),
   R¹ and R², R² and R³, or R¹ and R³ may be bonded to form a cyclic structure, and in this case, the cyclic structure is a 5- to 7-membered ring, and may further include a nitrogen atom, a sulfur atom or an oxygen atom, and when the cyclic structure has a double bond, it contains a nitrogen atom other than the N atom in General Formula (1), and
   in General Formula (1), the total number of primary, secondary or tertiary amino groups is greater than the total number of -COOH and -SO₃H) groups.
[3] The method according to [1] or [2], wherein the proton acceptor is tris(hydroxymethyl)aminomethane and/or 2-morpholinoethanesulfonic acid.
[4] The method according to any one of [1] to [3], wherein the sample is derived from at least one specimen selected from the group consisting of blood, cerebrospinal fluid, urine, feces, sputum, saliva, nasal discharge, swab fluid, amniotic fluid, and gargle fluid.
[5] The method according to any one of [1] to [4], wherein, in the contacting step, at least a portion of the nucleic acid is not adsorbed to the zeolite.
[6] The method according to any one of [1] to [5], wherein the zeolite is at least one selected from the group consisting of a ferrierite-type zeolite, a ZSM-5-type zeolite, a Y-type zeolite, a beta-type zeolite, and a mordenite type.
[7] The method according to any one of [1] to [6], wherein the zeolite is at least one selected from the group consisting of a ferrierite-NH₄-type zeolite, a ferrierite-K-type zeolite, a ZSM-5-NH₄-type zeolite, a Y-Na-type zeolite, a beta-NH₄-type zeolite, a mordenite-H-type zeolite, a mordenite-Na type, and a ferrierite-H-type zeolite.
[8] The method according to any one of [1] to [7], wherein the nucleic acid is RNA and/or DNA.
[9] A kit for purifying a nucleic acid, including a zeolite and a proton acceptor.
[10] The kit according to [9], wherein the proton acceptor is a compound represented by General Formula (1): 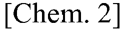 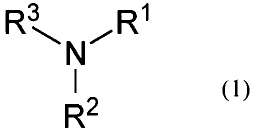 (in the formula, R¹, R² and R³ are each independently a hydrogen atom, an optionally substituted aliphatic hydrocarbon group having 1 to 6 carbon atoms, or an optionally substituted chain aliphatic hydrocarbon group having 2 to 10 carbon atoms and in which a nitrogen atom, a sulfur atom or an oxygen atom is interposed between carbon atoms, the substituent is selected from the group consisting of a hydroxyl group, a carboxyl group, an amino group, a sulfonic acid group, an imino group, a guanidyl group, an aromatic hydrocarbon group having 6 to 12 carbon atoms, and a 3- to 6-membered heterocyclic group which contains, as a ring-constituting atom, at least one atom selected from the group consisting of a nitrogen atom, a sulfur atom and an oxygen atom, and which may have a substituent that is an aliphatic hydrocarbon group having 1 to 6 carbon atoms,
   when the optionally substituted aliphatic hydrocarbon group having 1 to 6 carbon atoms has a substituent that is an aromatic hydrocarbon group having 6 to 12 carbon atoms, the aliphatic hydrocarbon group having 1 to 6 carbon atoms is an aliphatic hydrocarbon group having 2 to 6 carbon atoms, and the aromatic hydrocarbon group is not bonded to the carbon atom of the aliphatic hydrocarbon group having 2 to 6 carbon atoms that is closest to the N atom in General Formula (1),
   R¹ and R², R² and R³, or R¹ and R³ may be bonded to form a cyclic structure, and in this case, the cyclic structure is a 5- to 7-membered ring, and may further include a nitrogen atom, a sulfur atom or an oxygen atom, and when the cyclic structure has a double bond, it contains a nitrogen atom other than the N atom in General Formula (1), and
   in General Formula (1), the total number of primary, secondary or tertiary amino groups is greater than the total number of -COOH and -SO₃H groups).
[11] The kit according to [9] or [10], wherein the proton acceptor is tris(hydroxymethyl)aminomethane and/or 2-morpholinoethanesulfonic acid.
[12] The kit according to any one of [9] to [11], wherein the zeolite is at least one selected from the group consisting of a ferrierite-type zeolite, a ZSM-5-type zeolite, a Y-type zeolite, a beta-type zeolite, and a mordenite type.
[13] The kit according to any one of [9] to [12], wherein the zeolite is at least one selected from the group consisting of a ferrierite-NH₄-type zeolite, a ferrierite-K-type zeolite, a ZSM-5-NH₄-type zeolite, a Y-Na-type zeolite, a beta-NH₄-type zeolite, a mordenite-H-type zeolite, a mordenite-Na type, and a ferrierite-H-type zeolite.
[14] The kit according to any one of [9] to [13], wherein the kit is a kit for purifying a nucleic acid derived from at least one specimen selected from the group consisting of blood, cerebrospinal fluid, urine, feces, sputum, saliva, nasal discharge, swab fluid, amniotic fluid, and gargle fluid, and further includes a nucleic acid extraction reagent containing a surfactant.
[15] The kit according to any one of [9] to [14], wherein the nucleic acid is RNA and/or DNA.

### Advantageous Effects of Invention

According to the present invention, it is possible to provide a method of purifying a nucleic acid from a sample containing a nucleic acid easily, rapidly, and with high purity and a kit for such purification. Particularly, according to the present invention, it is possible to purify a nucleic acid from a sample containing a nucleic acid easily, rapidly, and with high purity without using a plurality of devices such as a high-speed centrifuge and a heating heater. In addition, the present invention is highly versatile because it can be applied to purification of either or both of DNA and RNA.

### Description of Embodiments

Hereinafter, forms for implementing the present invention will be described in detail. However, the present invention is not limited to the following embodiments.

### [Nucleic acid purification method]

A nucleic acid purification method according to the present embodiment includes a step of bringing a sample containing a nucleic acid into contact with a zeolite and a proton acceptor. The contacting step is a step of bringing a sample containing a nucleic acid into contact with a zeolite and a proton acceptor, and the zeolite adsorbs substances other than a nucleic acid. As will be shown in examples below, when a sample containing a nucleic acid is brought into contact with a zeolite and a proton acceptor, the effect of the zeolite adsorbing substances other than a nucleic acid is improved. Therefore, it is possible to purify a nucleic acid from the sample containing a nucleic acid easily, rapidly, and with high purity.

### <Sample containing nucleic acid>

The sample containing a nucleic acid is an aqueous solution or aqueous suspension in which a nucleic acid and substances other than a nucleic acid are dissolved or suspended. The type of a nucleic acid contained in the sample is not particularly limited, and may be DNA or RNA, and may be single-stranded or double-stranded. The origin of a nucleic acid is not limited, and for example, a nucleic acid may be derived from animals, plants, fungi, bacteria, or viruses. The method of the present invention can particularly purify RNA easily with high purity. The pH of the sample containing a nucleic acid is not particularly limited.

Examples of substances other than a nucleic acid include proteins (for example, enzymes, glycoproteins, polypeptides, etc.), lipids, sugars (for example, monosaccharides, disaccharides, oligosaccharides, polysaccharides, etc.), salts and the like.

In addition, the sample containing a nucleic acid may be derived from, for example, a specimen obtained from a living organism, or may be derived from a specimen obtained from the environment such as water, soil, or air. The sample containing a nucleic acid is preferably derived from a specimen obtained from a living organism, and more preferably derived from at least one specimen selected from the group consisting of blood (whole blood, plasma, or serum), cerebrospinal fluid, urine, feces, sputum, saliva, nasal discharge, swab fluid, amniotic fluid, and gargle fluid. The sample containing a nucleic acid may be a diluted solution/suspension of the specimen or an extraction solution obtained according to an extraction step described below. The substances other than a nucleic acid may be components other than a nucleic acid derived from these specimens.

The sample containing a nucleic acid may contain a nucleic acid extraction reagent including a surfactant to be described below, and may also contain, as necessary, an inhibitor against substances that decompose a nucleic acid. Examples of such inhibitors include a nuclease inhibitor.

### <Zeolite>

"Zeolite" is a general term for aluminosilicates having micropores in their crystals, and having a three-dimensional network structure as a basic framework, in which SiO₄ and AlO₄ tetrahedrons are bonded by sharing oxygen atoms. Zeolites contain cations in their crystals, for example, alkali metal ions, alkaline earth metal ions, ammonium ions, hydrogen ions (protons) and the like. "Proton-type zeolite" is a zeolite that contains hydrogen ions as cations in its crystal. "Aprotic zeolite" is a zeolite other than a proton-type zeolite. The zeolite in the purification method according to the present embodiment does not adsorb at least a portion of a nucleic acid in the sample containing a nucleic acid.

The crystal structure of the zeolite is not particularly limited, and examples thereof include A-type, ferrierite-type, MCM-22-type, ZSM-5-type, mordenite-type, L-type, Y-type, X-type, and beta-type. The zeolite is preferably at least one selected from the group consisting of a ferrierite-type zeolite, a ZSM-5-type zeolite, a Y-type zeolite, a beta-type zeolite, and a mordenite type.

The cations in the crystals of the zeolite are not particularly limited. Examples of such cations include alkali metal ions (for example, sodium ions (Na⁺), potassium ions (K⁺), etc.), alkaline earth metal ions (for example, magnesium ions (Mg²⁺), calcium ions (Ca²⁺), etc.), ammonium ions (NH₄⁺), and hydrogen ions (H⁺). The cations in the crystals of the zeolite are preferably other than hydrogen ions (H⁺). That is, the zeolite is preferably an aprotic zeolite.

Particularly, the zeolite is preferably at least one selected from the group consisting of ferrierite-NH₄-type zeolite, ferrierite-K-type zeolite, ZSM-5-NH₄-type zeolite, Y-Na-type zeolite, beta-NH₄-type zeolite, mordenite-H-type zeolite, mordenite-Na type, and ferrierite-H-type zeolite, and more preferably at least one selected from the group consisting of ferrierite-NH₄-type zeolite, ferrierite-K-type zeolite, ZSM-5-NH₄-type zeolite, Y-Na-type zeolite, beta-NH₄-type zeolite, and mordenite-Na type. For example, "ferrierite-NH₄-type zeolite" is a zeolite having a ferrierite-type crystal structure and containing ammonium ions as cations.

### <Proton acceptor>

In this specification, the "proton acceptor" is a compound that can accept protons (hydrogen ions) in a neutral aqueous solution. For example, it is a compound in which the number of moles of protons (H⁺) that can be accepted (via a coordinate bond) is greater than the number of moles of protons that can be donated (released). In other words, it may be a compound in which the number of moles of atoms or functional groups (proton-accepting groups) that accept protons is greater than the number of moles of atoms or functional groups (proton-donating groups) that donate protons. Proton-accepting groups (or electron donating groups) and proton-donating groups (or electron accepting groups) can be determined by those skilled in the art based on the structure of the functional group, examples of proton-accepting groups include a primary amino group, a secondary amino group, and a tertiary amino group, and examples of proton-donating groups include functional groups such as a carboxyl group (-COOH) and a sulfonic acid group (-SO₃H). For example, tris(hydroxymethyl)aminomethane has one amino group that is a proton-accepting group and has no proton-donating group. Therefore, the number of moles of protons that can be accepted is greater than the number of moles of protons that can be donated throughout the compound, and it can be said to be a proton acceptor. On the other hand, tricine has one amino group that is a proton-accepting group and one carboxyl group that is a proton-donating group. Therefore, the number of moles of protons that can be accepted and the number of moles of protons that can be donated are the same throughout the compound, and thus it cannot be said to be a proton acceptor. Here, in the structure of the compound, since the proton-accepting group and the proton-donating group may become less likely to accept or donate protons due to interactions (for example, resonance) with nearby functional groups, the proton acceptor may not be simply determined by the number of moles of proton-accepting groups and proton-donating groups. Therefore, in order to function as a proton-accepting group, a so-called "lone pair" is required.

The proton acceptor may be a compound represented by the following General Formula (1).

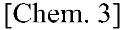

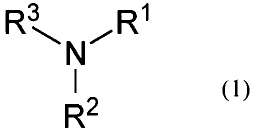

In the formula, R¹, R² and R³ are each independently a hydrogen atom, an optionally substituted aliphatic hydrocarbon group having 1 to 6 carbon atoms, or an optionally substituted chain aliphatic hydrocarbon group having 2 to 10 carbon atoms and in which a nitrogen atom, a sulfur atom or an oxygen atom is interposed between carbon atoms, the substituent is selected from the group consisting of a hydroxyl group, a carboxyl group, an amino group, a sulfonic acid group, an imino group, a guanidyl group, an aromatic hydrocarbon group having 6 to 12 carbon atoms, and a 3- to 6-membered heterocyclic group which contains, as a ring-constituting atom, at least one atom selected from the group consisting of a nitrogen atom, a sulfur atom and an oxygen atom, and which may have a substituent that is an aliphatic hydrocarbon group having 1 to 6 carbon atoms,
when the optionally substituted aliphatic hydrocarbon group having 1 to 6 carbon atoms has a substituent that is an aromatic hydrocarbon group having 6 to 12 carbon atoms, the aliphatic hydrocarbon group having 1 to 6 carbon atoms is an aliphatic hydrocarbon group having 2 to 6 carbon atoms, and the aromatic hydrocarbon group is not bonded to the carbon atom of the aliphatic hydrocarbon group having 2 to 6 carbon atoms that is closest to the N atom in General Formula (1),
R¹ and R², R² and R³, or R¹ and R³ may be bonded to form a cyclic structure, and in this case, the cyclic structure is a 5- to 7-membered ring, and may further include a nitrogen atom, a sulfur atom or an oxygen atom, and when the cyclic structure has a double bond, it contains a nitrogen atom other than the N atom in General Formula (1), and
in General Formula (1), the total number of primary, secondary or tertiary amino groups is greater than the total number of -COOH and -SO₃H groups.

At least one or at least two of R¹, R² and R³ may be a hydrogen atom, and all of R¹, R² and R³ may be a hydrogen atom.

When R¹, R² and R³ are each independently an optionally substituted aliphatic hydrocarbon group having 1 to 6 carbon atoms, the number of carbon atoms of the aliphatic hydrocarbon group may be 1 to 5, 1 to 4, 1 to 3, 2 or 1. The aliphatic hydrocarbon group may be a saturated hydrocarbon group or an unsaturated hydrocarbon group, or may be a chain hydrocarbon group or a cyclic hydrocarbon group. When the aliphatic hydrocarbon group is a cyclic hydrocarbon group, it may be an alicyclic hydrocarbon group or an aromatic hydrocarbon ring.

Examples of aliphatic hydrocarbon groups having 1 to 6 carbon atoms include an alkyl group having 1 to 6 carbon atoms, preferably 1 to 5 carbon atoms, an alkenyl group having 2 to 6 carbon atoms, preferably 2 to 5 carbon atoms, an alkynyl group having 2 to 6 carbon atoms, preferably 2 to 5 carbon atoms, a cycloalkyl group having 3 to 6 carbon atoms, preferably 3 to 5 carbon atoms, a cycloalkenyl group having 3 to 6 carbon atoms, preferably 3 to 5 carbon atoms, and a cycloalkynyl group having 3 to 6 carbon atoms, preferably 3 to 5 carbon atoms.

When R¹, R² and R³ are each independently an optionally substituted chain aliphatic hydrocarbon group having 2 to 10 carbon atoms in which a nitrogen atom, a sulfur atom or an oxygen atom is interposed between carbon atoms, the number of carbon atoms of the chain aliphatic hydrocarbon group may be 2 to 9, 3 to 9, 4 to 8, 5 to 7, or 6 or 7. The aliphatic hydrocarbon group in which a nitrogen atom, a sulfur atom, or an oxygen atom is interposed between carbon atoms is, for example, one in which, between two carbon atoms in the aliphatic hydrocarbon group, there is a nitrogen atom (-NH-, -NR-, or =N-), a sulfur atom (-S-), or an oxygen atom (-O-) covalently bonded to each of the carbon atoms. The number of nitrogen atoms, sulfur atoms or oxygen atoms interposed between carbon atoms may be one, two, or three, and one, two, or three types of nitrogen atoms, sulfur atoms and oxygen atoms may be present. The chain aliphatic hydrocarbon group may be a linear hydrocarbon group or a branched hydrocarbon group.

Examples of chain aliphatic hydrocarbon groups having 2 to 10 carbon atoms in which a nitrogen atom, a sulfur atom, or an oxygen atom is interposed between carbon atoms include functional groups in which a nitrogen atom, a sulfur atom, or an oxygen atom is interposed between carbon atoms in an alkyl group having 2 to 10 carbon atoms, preferably 3 to 10, 4 to 10, 5 to 10, or 6 to 10 carbon atoms, an alkenyl group having 2 to 10 carbon atoms, preferably 3 to 10, 4 to 10, 5 to 10, or 6 to 10 carbon atoms, an alkynyl group having 2 to 10 carbon atoms, preferably 3 to 10, 4 to 10, 5 to 10, or 6 to 10 carbon atoms, a cycloalkyl group having 3 to 10 carbon atoms, preferably 4 to 10, 5 to 10, or 6 to 10 carbon atoms, a cycloalkenyl group having 3 to 10 carbon atoms, preferably 4 to 10, 5 to 10, or 6 to 10 carbon atoms, a cycloalkynyl group having 3 to 10 carbon atoms, preferably 4 to 10, 5 to 10, or 6 to 10 carbon atoms, and the like.

The substituent is selected from the group consisting of a hydroxyl group, a carboxyl group, an amino group, a sulfonic acid group, an imino group, a guanidyl group, an aromatic hydrocarbon group having 6 to 12 carbon atoms, and a 3- to 6-membered heterocyclic group which contains, as a ring-constituting atom, at least one atom selected from the group consisting of a nitrogen atom, a sulfur atom and an oxygen atom, and which may have a substituent that is an aliphatic hydrocarbon group having 1 to 6 carbon atoms. The aromatic hydrocarbon group having 6 to 12 carbon atoms may be a monocyclic aromatic hydrocarbon or a polycyclic aromatic hydrocarbon group, the aromatic hydrocarbon group may be, for example, an aromatic hydrocarbon group having 6 to 12 carbon atoms, and preferably 6 to 10, or 6 to 8 carbon atoms, and examples thereof include a phenyl group, a toluyl group, and a naphthyl group. The 3- to 6-membered heterocyclic group which contains, as a ring-constituting atom, at least one atom selected from the group consisting of a nitrogen atom, a sulfur atom and an oxygen atom, and which may have a substituent that is an aliphatic hydrocarbon group having 1 to 6 carbon atoms is, for example, an imidazole group.

When the optionally substituted aliphatic hydrocarbon group having 1 to 6 carbon atoms has a substituent that is an aromatic hydrocarbon group having 6 to 12 carbon atoms, the aliphatic hydrocarbon group having 1 to 6 carbon atoms is an aliphatic hydrocarbon group having 2 to 6 carbon atoms, and the aromatic hydrocarbon group is not bonded to the carbon atom of the aliphatic hydrocarbon group having 2 to 6 carbon atoms that is closest to the N atom in General Formula (1). In this case, since the aromatic hydrocarbon group having 6 to 12 carbon atoms does not resonate with the N atom in General Formula (1), the N atom in General Formula (1) has a lone pair.

R¹ and R², R² and R³, or R¹ and R³ may be bonded to form a cyclic structure, and in this case, the cyclic structure is a 5- to 7-membered ring, and may further include a nitrogen atom, a sulfur atom or an oxygen atom, and when the cyclic structure has a double bond, it contains a nitrogen atom other than the N atom in General Formula (1). When the cyclic structure has a double bond, since the double bond may resonate with the N atom in General Formula (1), the N atom in General Formula (1) may not have a lone pair. However, when the cyclic structure has a nitrogen atom other than the N atom in General Formula (1), that is, a proton-accepting group other than the N atom in General Formula (1), the compound of General Formula (1) may be a proton acceptor. Examples of such compounds include imidazole. R¹ and R², R² and R³, or R¹ and R³ are bonded to form a ring, for example, via a single bond or double bond between two carbon atoms derived from R¹, R² or R³, a single bond between a carbon atom and an oxygen atom or a sulfur atom, or a single bond or double bond between a carbon atom and a nitrogen atom. Examples of 5- to 7-membered cyclic structures include an imidazole ring, a pyrrolidine ring, a piperazine ring, a piperidine ring, a thiomorpholine ring, and a morpholine ring. Specific examples of compounds include imidazole or derivatives thereof, pyrrolidine or derivatives thereof, piperazine or derivatives thereof, piperidine or derivatives thereof, thiomorpholine or derivatives thereof, and morpholine or derivatives thereof.

In General Formula (1), the total number of primary, secondary or tertiary amino groups is greater than the total number of -COOH and -SO₃H groups, and may be, for example, one or more greater, two or more greater, or three or more greater. Since the primary, secondary or tertiary amino groups are proton-accepting groups whereas -COOH and -SO₃H are proton-donating groups, when the total number of primary, secondary or tertiary amino groups is greater than the total number of - COOH and -SO₃H groups, the entire compound functions as a proton acceptor.

As described above, the compound represented by General Formula (1) is a proton acceptor, and in terms of the chemical structure, it includes some amines or derivatives thereof, some amino acids or derivatives thereof, and the like. The amines may be, for example, primary amines, secondary amines, or tertiary amines, and may be aliphatic amines, aromatic amines, or heterocyclic amines.

Examples of aliphatic amines, which are compounds represented by General Formula (1), include tris(hydroxymethyl)aminomethane, tert-butylamine, ethylamine, isopropylamine, ethanolamine, 1,3-bis[tris(hydroxymethyl)methylamino]propane, diethylamine, trimethylamine, triethylamine, bis(2-hydroxyethyl)iminotris(hydroxymethyl)methane (bis-tris), arginine, and lysine.

Examples of heterocyclic amines, which are compounds represented by General Formula (1), include imidazole or derivatives thereof, pyrrolidine or derivatives thereof, piperazine or derivatives thereof, piperidine or derivatives thereof, thiomorpholine or derivatives thereof, morpholine or derivatives thereof, and histidine.

Examples of imidazole derivatives include 1-methylimidazole, 2-methylimidazole, 4-methylimidazole, 1-ethylimidazole, 2-ethylimidazole, 1-propylimidazole, 2-propylimidazole, 1-isopropylimidazole, 2-isopropylimidazole, 1-butylimidazole, 2-butylimidazole, 1-tert-butylimidazole, 2-tert-butylimidazole, 1,2-dimethylimidazole, 2-ethyl 4-methylimidazole, 2-hydroxymethyl-1-methylimidazole, 4-hydroxymethyl-5-methylimidazole, 2-(1H-imidazole-1-yl)ethanol, 2-mercapto-1-methylimidazole, and 1,2,4,5-tetramethylimidazole.

Examples of piperazine derivatives include 1,4-dimethylpiperazine, 2-methylpiperazine, 1-ethylpiperazine(N-ethylpiperazine), 2,5-dimethylpiperazine, 2,6-dimethylpiperazine, 1-amino-4-methylpiperazine, 1-acetylpiperazine, 1-(2-aminoethyl)piperazine, 1-(2-hydroxyethyl)piperazine, 1-butylpiperazine, 1-(methylsulfonyl)piperazine, 1,4-bis(2-hydroxyethyl)piperazine, 1-(2-hydroxyethyl)piperazine-4-(2-ethanesulfonic acid), 1-(2-hydroxyethyl)piperazine-4-(3-propanesulfonic acid), and 1-(2-hydroxyethyl)piperazine-4-(2-hydroxy-3-propanesulfonic acid).

Examples of piperidine derivatives include 1-methylpiperidine, 2-methylpiperidine, 3-methylpiperidine, 4-methylpiperidine, 1-aminopiperidine, 4-aminopiperidine, 3-hydroxypiperidine, 4-hydroxypiperidine, 2-ethylpiperidine, 2,6-dimethylpiperidine, ethyl 3-piperidinecarboxylate, and ethyl 2-piperidinecarboxylate.

Examples of pyrrolidine derivatives include 1-methylpyrrolidine, 2-methylpyrrolidine, 3-aminopyrrolidine, 3-hydroxypyrrolidine, 1-ethylpyrrolidine, 3-hydroxy-1-methylpyrrolidine, 3-methoxypyrrolidine, (S)-(+)-2-(hydroxymethyl)pyrrolidine, 3-hydroxymethylpyrrolidine, 3-(dimethylamino)pyrrolidine, and 1-ethyl-3-pyrrolidinol.

Examples of thiomorpholine derivatives include thiomorpholine 1,1-dioxide, and 4-aminothiomorpholine 1,1-dioxide.

Examples of morpholine derivatives include 4-methylmorpholine, 2-methylmorpholine, 4-aminomorpholine (N-aminomorpholine), 4-ethylmorpholine, 2,6-dimethylmorpholine, 4-(2-aminoethyl)morpholine, 4-(2-hydroxyethyl)morpholine, 2-morpholinoethanesulfonic acid, and 3-morpholinepropanesulfonic acid.

### <Contacting step>

In the contacting step, a sample containing a nucleic acid is brought into contact with a zeolite and a proton acceptor to adsorb substances other than a nucleic acid. Therefore, in the purification method according to the present embodiment, it is possible to purify a nucleic acid from the sample containing a nucleic acid according to the contacting step. "Purification" in this specification means increasing the purity of a nucleic acid from a sample containing a nucleic acid.

In the contacting step, "bring into contact" may mean mixing a sample containing a nucleic acid, a zeolite and a proton acceptor, and for example, a sample containing a nucleic acid may be added to a container containing a zeolite, a zeolite may be added to a sample containing a nucleic acid, and the order in which a sample containing a nucleic acid, a zeolite, and a proton acceptor are brought into contact with each other is not particularly limited.

In the contacting step, the amount of the zeolite per 1 mL of the sample containing a nucleic acid may be, for example, 50 mg or more, 60 mg or more, 70 mg or more, 80 mg or more, 90 mg or more, 100 mg or more, 110 mg or more, or 120 mg or more. In the contacting step, the amount of the zeolite per 1 mL of the sample containing a nucleic acid may be, for example, 350 mg or less, 340 mg or less, 330 mg or less, 320 mg or less, 310 mg or less, or 300 mg or less.

In the contacting step, the amount of the proton acceptor may vary depending on the proton-accepting capacity (in molar equivalents) of the compound, and for example, in the case of tris(hydroxymethyl)aminomethane (Tris), the amount of the proton acceptor with respect to a total amount of the sample containing a nucleic acid may be 10 mM or more, 12 mM or more, 14 mM or more, 16 mM or more, 18 mM or more, 20 mM or more, 22 mM or more, 24 mM or more, or 25 mM or more. In the contacting step, the content of Tris with respect to a total amount of the sample containing a nucleic acid may be, for example, 300 mM or less, 250 mM or less, 200 mM or less, 150 mM or less, 100 mM or less, or 80 mM or less.

In the contacting step, the contact time is not particularly limited as long as the sample containing a nucleic acid is brought into sufficient contact with the zeolite and the proton acceptor, and may be, for example, 10 seconds to 10 minutes, 20 seconds to 10 minutes, or 30 seconds to 5 minutes.

According to the contacting step, at least some substances other than a nucleic acid in the sample containing a nucleic acid are adsorbed to the zeolite. "Adsorption" refers to the state in which a substance is bound to the surface or entrapped within the pores of the zeolite, and the adsorbed substance is removed together when the zeolite is removed. According to the contacting step, at least 30% or more, at least 40% or more, at least 50% or more, at least 60% or more, at least 70% or more, at least 80% or more, or at least 90% or more of substances other than a nucleic acid in the sample containing a nucleic acid are adsorbed to the zeolite.

### <Extraction step>

The purification method according to the present embodiment may include, before the contacting step, a step of obtaining a sample containing the nucleic acid by mixing the specimen with a nucleic acid extraction reagent containing a surfactant and/or by heating the specimen (extraction step). Due to the surfactant or the heat treatment, cell membranes, virus envelopes and the like in the specimen are destroyed. Therefore, the nucleic acid can be dissolved or suspended in the sample containing a nucleic acid. When both mixing the specimen with the nucleic acid extraction reagent containing a surfactant and heating the specimen are performed, the order of these steps is not particularly limited. When the purification method according to the present embodiment includes the extraction step, the purification method according to the present embodiment can be considered as an extraction method.

The surfactant is not particularly limited, and may be an ionic surfactant (anionic surfactant, cationic surfactant), a nonionic surfactant, or an amphoteric surfactant, and in order to more easily destroy cell membranes, virus envelopes and the like, an ionic surfactant is preferable, and an anionic surfactant is more preferable. The nucleic acid extraction reagent may contain one type of surfactant or two or more types of surfactants.

Examples of anionic surfactants include higher fatty acid salts, linear alkylbenzene sulfonates, α-sulfo fatty acid methyl ester salts, α-olefin sulfonates, alkylsulfate ester salts, and polyoxyethylene alkylsulfate ester salts, and alkylsulfate ester salts are preferable, and lithium dodecyl sulfate (LDS), sodium dodecyl sulfate (SDS), and sodium cholate are more preferable.

Examples of cationic surfactants include alkyltrimethylammonium salts, dialkyldimethylammonium salts, alkyldimethylbenzylammonium salts, and benzyltrimethylammonium salts, and may include benzalkonium chloride and the like.

Examples of nonionic surfactants include sorbitan fatty acid esters, sucrose fatty acid esters, polyoxyethylene fatty acid esters, polyoxyethylene sorbitan fatty acid esters, polyoxyethylene alkyl ethers, and polyoxyalkylphenyl ethers, and may include TRITON (registered trademark)-X100, Tween (registered trademark) 40 and the like.

Examples of amphoteric surfactants include alkylamino fatty acid salts, alkyl betaines, and alkyl amine oxides, and may include 3-[(3-cholamidopropyl)dimethylammonio]-2-hydroxy-1-propanesulfon ate (CHAPSO), 3-[(3-cholamidopropyl)dimethylammonio]-1-propanesulfonate (CHAPS) and the like.

The content of the surfactant in the nucleic acid extraction reagent may be a general content that allows a nucleic acid to be extracted, and may vary depending on the type of surfactant. The content of the surfactant based on a total amount of the nucleic acid extraction reagent may be, for example, 0.01 w/v% or more, 0.1 w/v% or more, 0.5 w/v% or more, or 1 w/v% or more. The content of the surfactant in the nucleic acid extraction reagent based on a total amount of the nucleic acid extraction reagent may be, for example, 5 w/v% or less, 4 w/v% or less, or 3 w/v% or less. Here, when the nucleic acid extraction reagent contains a plurality of types of surfactants, the content of the surfactant in the nucleic acid extraction reagent is the total content of the plurality of types of surfactants.

The nucleic acid extraction reagent may contain an alkali in addition to the surfactant. Examples of alkalis include sodium hydroxide, potassium hydroxide, lithium hydroxide, calcium hydroxide, and aqueous ammonia, and sodium hydroxide is preferable. The nucleic acid extraction reagent may contain one type of alkali or two or more types of alkali.

When the alkali is sodium hydroxide, the content of the alkali in the nucleic acid extraction reagent based on a total amount of the nucleic acid extraction reagent may be, for example, 10 mM or more, 25 mM or more, 50 mM or more, 75 mM or more, 100 mM or more, 125 mM or more, 150 mM or more, 175 mM or more, or 200 mM or more. When the alkali is sodium hydroxide, the content of the alkali in the nucleic acid-containing reagent based on a total amount of the nucleic acid extraction reagent may be, for example, 500 mM or less, 480 mM or less, 460 mM or less, 440 mM or less, 420 mM or less, or 400 mM or less.

The pH of the nucleic acid extraction reagent is not particularly limited as long as it is a general pH at which a nucleic acid does not decompose, and may be, for example, 5 to 9, 5 to 7, or 6 to 7.

The heat treatment is not particularly limited as long as a nucleic acid can be extracted from a specimen and the nucleic acid can be dissolved or suspended in a sample containing a nucleic acid, and for example, a specimen may be heated using a heat block or the like. The heat treatment temperature may be, for example, 50°C to 200°C, 60°C to 150°C, or 70°C to 100°C. The heat treatment time may be, for example, 1 minute to 30 minutes, 5 minutes to 30 minutes, or 15 minutes to 30 minutes.

### <Removal step>

The purification method according to the present embodiment may include a step of removing the zeolite (removal step) after the contacting step. In the removal step, the zeolite is removed to obtain a sample containing a purified nucleic acid.

The zeolite can be removed, for example, by subjecting the mixture of the sample containing a nucleic acid, the zeolite and the proton acceptor after the contacting step to centrifugation, filtration, or a combination thereof. Removal by centrifugation can be easily performed using, for example, a commercially available spin column.

The sample containing a purified nucleic acid obtained in the removal step can be used, for example, in nucleic acid amplification analysis.

### [Nucleic acid amplification method]

A nucleic acid amplification method according to the present embodiment includes a step of purifying a nucleic acid by performing the purification method according to the present embodiment, and a step of performing a nucleic acid amplification reaction on the purified nucleic acid (nucleic acid amplification step).

### <Nucleic acid amplification step>

The nucleic acid amplification step can be performed according to a conventional method. More specifically, it can be performed by incubating a reaction solution containing the purified nucleic acid, a primer set containing a base sequence specific to the nucleic acid, a nucleic acid synthesis enzyme such as a DNA polymerase, deoxynucleoside triphosphates (dNTPs: dATP, dTTP, dCTP, and dGTP), a reverse transcriptase as necessary, a labeled probe for detecting an amplification product and the like.

When the nucleic acid is DNA, the nucleic acid amplification reaction may be, for example, a polymerase chain reaction (PCR), a loop-mediated isothermal amplification (LAMP) reaction, a nicking endonuclease amplification reaction (NEAR), a transcription-reverse transcription concerted (TRC) reaction, a whole genome amplification (WGA) reaction, a strand displacement amplification (SDA) reaction, a helicase-dependent amplification (HDA) reaction, a recombinase polymerase amplification (RPA) reaction, or an isothermal chimeric primer-initiated amplification (ICAN) reaction. When the nucleic acid is RNA, the nucleic acid amplification reaction may be a reverse transcription PCR (RT-PCR), a reverse transcription LAMP (RT-LAMP) reaction, a transcription-mediated amplification (TMA) reaction, or a nucleic acid sequence-based amplification (NASBA) reaction.

### [Kit for purifying nucleic acid]

A kit for purifying a nucleic acid according to the present embodiment (hereinafter referred to as a "purification kit according to the present embodiment") includes a zeolite and a proton acceptor. When the purification kit according to the present embodiment is used, it is possible to purify a nucleic acid easily and with high purity from the sample containing a nucleic acid. The sample containing a nucleic acid, the zeolite, and the proton acceptor are as described above.

The purification kit according to the present embodiment may include a nucleic acid extraction reagent containing a surfactant when a specimen-derived sample is used as the sample containing a nucleic acid. The nucleic acid extraction reagent containing a surfactant is as described above.

The purification kit according to the present embodiment may include, in addition to the zeolite, the proton acceptor, and the nucleic acid extraction reagent containing a surfactant, a buffer solution, a nuclease inhibitor, a device used for purifying a nucleic acid (for example, a spin column, etc.) and the like.

### [Kit for nucleic acid amplification]

A kit for nucleic acid amplification according to the present embodiment (hereinafter referred to as a "nucleic acid amplification kit according to the present embodiment") includes a zeolite, a proton acceptor, a DNA polymerase, and deoxynucleoside triphosphates.

The nucleic acid amplification kit according to the present embodiment may include, in addition to the components provided in the purification kit according to the present embodiment, known reagents for performing various nucleic acid amplification reactions. When the target nucleic acid is RNA, the nucleic acid amplification kit according to the present embodiment may further include a reverse transcriptase. Examples of other known reagents for performing various nucleic acid amplification reactions include a buffer solution that provides conditions suitable for enzymatic reactions, enzymes such as dithiothreitol (DTT), protecting agents for stabilizing target nucleic acid sequences, labeled probes for detecting amplification products, and intercalators. In addition, the nucleic acid amplification kit according to the present embodiment may include a device used for detecting nucleic acid amplification.

### [Examples]

Hereinafter, the present invention will be described in more detail with reference to examples. However, the present invention is not limited thereto.

### [Test Example 1: Evaluation 1 of effect of zeolite adsorbing substances other than nucleic acids]

The zeolite used was a ferrierite-NH₄-type zeolite (model number:720NHA, commercially available from Tosoh Corporation). Two types of nucleic acid extraction reagents were used: 0.5% sodium dodecyl sulfate (SDS) and 0.5% SDS+50 mM tris(hydroxymethyl)aminomethane buffer solution (Tris-HCl) (pH 7.0). The target nucleic acid solution used was SARS-CoV-2 transcription RNA (250 copies/test).

200 µL of a saliva specimen was added to 800 µL of a nucleic acid extraction reagent and mixed, 240 mg of a zeolite (720NHA) was then added, and additionally mixed, the mixture was then passed through a 0.45 µm filter, and the zeolite and substances adsorbed thereon were removed. The obtained filtrates were used as zeolite-treated samples 1-3 (only SDS) and 1-6 (SDS+Tris-HCl). To 9.5 µL of a zeolite-treated sample, 0.5 µL of a target nucleic acid solution was added, the mixture was reacted using a Loopamp (registered trademark) Novel Coronavirus 2019 (SARS-CoV-2) detection reagent kit (commercially available from Eiken Chemical Co., Ltd.), at 62.5°C for 35 minutes, and the target nucleic acid was amplified. The turbidity value, which increases as a result of target nucleic acid amplification (cDNA of SARS-CoV-2 transcription RNA), was measured in real time using a LoopampEXIA Amplification Unit (commercially available from Eiken Chemical Co., Ltd.).

As positive controls, samples 1-1 (only SDS) and 1-4 (SDS+Tris-HCl) obtained by adding distilled water (DW) to a nucleic acid extraction reagent in place of the saliva specimen, and mixing them were used. In addition, as negative controls, zeolite-untreated samples 1-2 (only SDS) and 1-5 (SDS+Tris-HCl) obtained without adding a zeolite were used. These samples were also subjected to target nucleic acid amplification according to an LAMP method in the same manner as above. The results are shown in Table 1. In Table 1, samples in which no detection occurred within the measurement time (35 minutes) are indicated as "N. D.".

**[Table 1]**

| Sample No. | Presence of buffer | Specimen | Zeolite treatment | Detection time (min) |
|---|---|---|---|---|
| 1-1 | no | DW | no | 18.9 |
| 1-2 | no | saliva | no | N.D. |
| 1-3 | no | saliva | 720NHA | N.D. |
| 1-4 | Tris-HCl | DW | no | 19.4 |
| 1-5 | Tris-HCl | saliva | no | N.D. |
| 1-6 | Tris-HCl | saliva | 720NHA | 19.6 |

In the samples containing no saliva (positive controls), regardless of the presence or absence of the buffer, target nucleic acid amplification was confirmed, and the detection time was also comparable (samples 1-1 and 1-4). This confirmed that the presence or absence of the buffer did not affect the detection time for the target nucleic acid. On the other hand, in the zeolite-untreated samples containing a saliva specimen (negative controls), regardless of the presence or absence of the buffer, target nucleic acid amplification was not confirmed (samples 1-2 and 1-5). This suggests that nucleic acids could not be purified using the buffer alone without using the zeolite. In addition, in the zeolite-treated samples containing a saliva specimen (samples 1-3 and 1-6), when the nucleic acid extraction reagent containing Tris-HCl (sample 1-6) was used, target nucleic acid amplification was confirmed, and the detection time was comparable to that of the positive control. On the other hand, when the nucleic acid extraction reagent containing no Tris-HCl (sample 1-3) was used, target nucleic acid amplification was not confirmed. These results suggest that, in the presence of Tris-HCl, the zeolite adsorbed substances other than nucleic acids in the sample and was able to remove these substances. That is, these results suggest, when the sample containing nucleic acids was brought into contact with the zeolite and Tris-HCl, it was possible to purify nucleic acids from the sample containing nucleic acids easily, rapidly, and with high purity.

### [Test Example 2: Evaluation 2 of effect of zeolite adsorbing substances other than nucleic acids]

Various samples were prepared in the same manner as in Test Example 1 except that three types of nucleic acid extraction reagents were used: 0.5% SDS without a buffer, 0.5% SDS+50 mM Tris-HCl (pH 7.0), and 0.5% SDS+50 mM tricine (pH 7.0). The obtained samples were reacted using a Loopamp (registered trademark) Novel Coronavirus 2019 (SARS-CoV-2) detection reagent kit (commercially available from Eiken Chemical Co., Ltd.), at 62.5°C for 35 minutes, the target nucleic acid (cDNA of SARS-CoV-2 transcription RNA) was amplified, and the turbidity value, which increases as a result of target nucleic acid amplification, was measured in real time using a LoopampEXIA Amplification Unit (commercially available from Eiken Chemical Co., Ltd.). The results are shown in Table 2. In Table 2, samples in which no detection occurred within the measurement time (35 minutes) are indicated as "N.D.".

**[Table 2]**

| Sample No. | Specimen | Zeolite treatment | Buffer | Detection time (min) |
|---|---|---|---|---|
| 2-1 | DW | no | no | 18.8 |
| 2-2 | DW | no | Tris-HCl | 19.3 |
| 2-3 | DW | no | tricine | 18.7 |
| 2-4 | saliva | no | no | N.D. |
| 2-5 | saliva | no | Tris-HCl | N.D. |
| 2-6 | saliva | no | tricine | N.D. |
| 2-7 | saliva | 720NHA | no | 26.9 |
| 2-8 | saliva | 720NHA | Tris-HCl | 21.7 |
| 2-9 | saliva | 720NHA | tricine | 27.4 |

In the sample containing no saliva, target nucleic acid amplification was confirmed even without the zeolite treatment (samples 2-1, 2-2, and 2-3). Nucleic acid amplification was hardly affected by the presence or absence or type of the buffer. On the other hand, in the zeolite-untreated sample containing a saliva specimen, regardless of the presence or absence of the buffer, target nucleic acid amplification was not confirmed (samples 2-4, 2-5, and 2-6). In addition, in the zeolite-treated samples containing a saliva specimen (samples 2-7, 2-8, and 2-9), when the nucleic acid extraction reagent containing Tris-HCl (sample 2-8) was used, target nucleic acid amplification was confirmed, and the detection time was faster than that of the sample 2-7 without the buffer and was comparable to that of the sample 2-2 containing no saliva specimen. On the other hand, when the nucleic acid extraction reagent containing tricine (sample 2-9) was used, target nucleic acid amplification was confirmed, but the detection time was clearly slower than that of the sample 2-3 containing no saliva specimen and was comparable to that of the sample 2-7 without the buffer. These results indicate that the effect of the zeolite adsorbing substances other than nucleic acids in the sample was promoted in the presence of Tris-HCl, but this promotion effect was not observed with tricine having a similar buffering effect (pH adjusting effect) to Tris-HCl. That is, it suggests that the promotion effect of Tris-HCl is not simply due to its pH adjusting effect.

### [Test Example 3: Effect of compounds on adsorption of substances other than nucleic acids by zeolite]

As nucleic acid extraction reagents, in addition to 0.5% SDS, and those obtained by adding compounds shown in the following Table 3 to 0.5% SDS to achieve a concentration of 50 mM and adjusting the pH to 7.0 were used. 200 µL of a saliva specimen was added to 800 µL of each nucleic acid extraction reagent and mixed, 240 mg of a ferrierite-NH₄-type zeolite (model number:720NHA, commercially available from Tosoh Corporation) was then added, and additionally mixed, the mixture was then passed through a 0.45 µm filter, and the zeolite and substances adsorbed thereon were removed. The obtained filtrates were used as zeolite-treated samples 3-3 to 3-40.Similarly, as positive controls, samples 3-1 and 3-2 in which distilled water was added in place of the saliva specimen were prepared, and as negative controls, zeolite-untreated samples 3-41 to 3-42 obtained without adding a zeolite were also prepared. In the same manner as in Test Example 1, 0.5 µL of the target nucleic acid solution SARS-CoV-2 transcription RNA (250 copies/test) was added to 9.5 µL of each of the obtained samples, the mixture was reacted using a Loopamp (registered trademark) Novel Coronavirus 2019 (SARS-CoV-2) detection reagent kit (commercially available from Eiken Chemical Co., Ltd.) at 62.5°C for 35 minutes, the target nucleic acid was amplified, and the turbidity value, which increases according to target nucleic acid (cDNA of SARS-CoV-2 transcription RNA) amplification, was measured in real time using a LoopampEXIA Amplification Unit (commercially available from Eiken Chemical Co., Ltd.). The results are shown in Table 4. In Table 4, samples in which no detection occurred within the measurement time (35 minutes) are indicated as "N.D.".

In the samples containing no saliva 3-1 and 3-2, even without the zeolite, target nucleic acid amplification was confirmed. In addition, when Tris was used alone without the zeolite treatment, there was almost no difference in the detection time compared to when Tris was not present.

Here, it was confirmed that no reaction promotion or reaction delay caused by the compounds occurred in the zeolite-untreated samples using any type of nucleic acid extraction reagent (some data not shown). Here, in the zeolite-untreated samples containing a saliva specimen, regardless of which nucleic acid extraction reagent was used, nucleic acid amplification (turbidity) was not detected within the measurement time (some data not shown).

**[Table 3]**

| Compound | Structural formula |
|---|---|
| Tris(hydroxymethyl)aminomethane (Tris) | 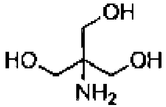 |
| tert-Butylamine | 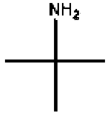 |
| Ethylamine | 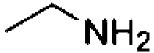 |
| Isopropylamine | 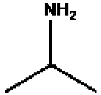 |
| Ethanolamine | 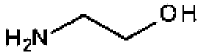 |
| 1,3-Bis[tris(hydroxymethyl)methylamino]prop ane | 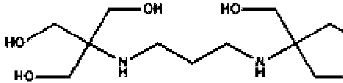 |
| Diethylamine | 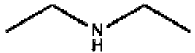 |
| Trimethylamine | 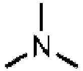 |
| Triethylamine | 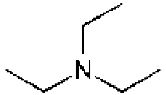 |
| Bis(2-hydroxyethyl)imino-tris(hydroxymethyl) methane (Bis-tris) | 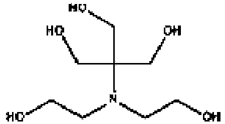 |
| Aniline | 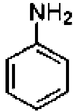 |
| Imidazole | 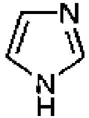 |
| 1,4-Dimethylpiperazine | 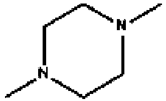 |
| 1-Methylpiperidine | 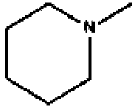 |
| Pyrrolidine | 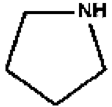 |
| 1-Methylpyrrolidine | 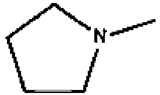 |
| Piperidine | 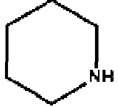 |
| Thiomorpholine | 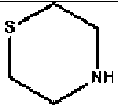 |
| Pyrrole | 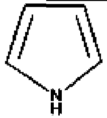 |
| Morpholine | 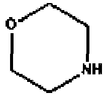 |
| 4-Methylmorpholine | 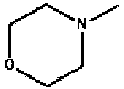 |
| 2-Morpholinoethanesulfonic acid | 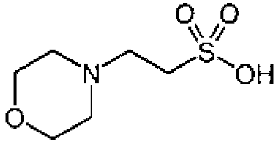 |
| 3-Morpholinopropanesulfonic acid | 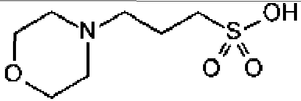 |
| L(+)-Arginine | 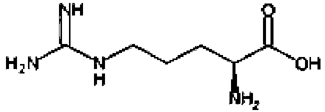 |
| L(+)-Lysine monohydrochloride | 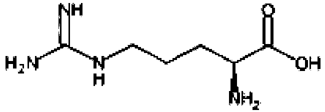 |
| L-Histidine monohydrochloride monohydrate | 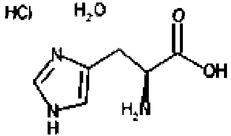 |
| L-Cysteine | 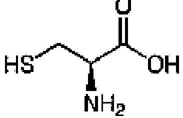 |
| Glycine | 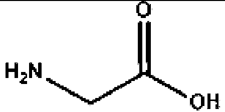 |
| L(-)-Proline | 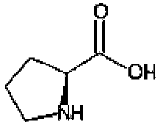 |
| L-Serine | 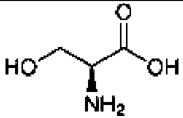 |
| Tricine | 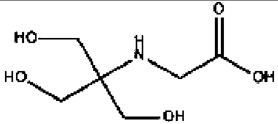 |
| Bicine | 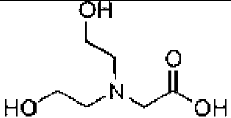 |
| 1,1,1-tris(hydroxymethyl)ethane | 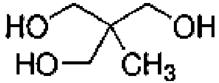 |
| Tetrahydropyran | 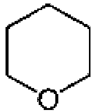 |
| Tetrahydrofuran | 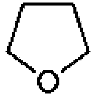 |
| 1,3-Dioxolane | 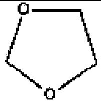 |
| 1,4-Dioxane | 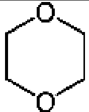 |

**[Table 4]**

| Sample No. | Speci men | Zeolite treatment | Compound | Detection time (min) |
|---|---|---|---|---|
| 3-1 | DW | no | no | 16.2 |
| 3-2 | DW | no | Tris | 16.9 |
| 3-3 | saliva | 720NHA | no | 22.1 |
| 3-4 | saliva | 720NHA | Tris | 16.9 |
| 3-5 | saliva | 720NHA | tert-Butylamine | 16.1 |
| 3-6 | saliva | 720NHA | Ethylamine | 16.8 |
| 3-7 | saliva | 720NHA | Isopropylamine | 16.3 |
| 3-8 | saliva | 720NHA | Ethanolamine | 16.3 |
| 3-9 | saliva | 720NHA | 1,3-Bis[tris(hydroxymethyl)methyl amino]propane | 16.1 |
| 3-10 | saliva | 720NHA | Diethylamine | 16.8 |
| 3-11 | saliva | 720NHA | Trimethylamine | 16.6 |
| 3-12 | saliva | 720NHA | Triethylamine | 16.3 |
| 3-13 | saliva | 720NHA | Bis-tris | 18.2 |
| 3-14 | saliva | 720NHA | Aniline | 24.6 |
| 3-15 | saliva | 720NHA | Imidazole | 16.5 |
| 3-16 | saliva | 720NHA | 1,4-Dimethylpiperazine | 17.4 |
| 3-17 | saliva | 720NHA | 1-Methylpiperidine | 16.9 |
| 3-18 | saliva | 720NHA | Pyrrolidine | 19.3 |
| 3-19 | saliva | 720NHA | 1-Methylpyrrolidine | 18.1 |
| 3-20 | saliva | 720NHA | Piperidine | 18.9 |
| 3-21 | saliva | 720NHA | Thiomorpholine | 19.2 |
| 3-22 | saliva | 720NHA | Pyrrole | 27.5 |
| 3-23 | saliva | 720NHA | Morpholine | 17.0 |
| 3-24 | saliva | 720NHA | 4-Methylmorpholine | 16.4 |
| 3-25 | saliva | 720NHA | 2-Morpholinoethanesulfonic acid | 18.9 |
| 3-26 | saliva | 720NHA | 3-Morpholinopropanesulfonic acid | 19.1 |
| 3-27 | saliva | 720NHA | Arginine | 20.2 |
| 3-28 | saliva | 720NHA | Lysine monohydrochloride | 19.2 |
| 3-29 | saliva | 720NHA | L-Histidine monohydrochloride monohydrate | 20.8 |
| 3-30 | saliva | 720NHA | Cysteine | 22.5 |
| 3-31 | saliva | 720NHA | Glycine | N.D. |
| 3-32 | saliva | 720NHA | Proline | N.D. |
| 3-33 | saliva | 720NHA | Serine | N.D. |
| 3-34 | saliva | 720NHA | Tricine | 27.4 |
| 3-35 | saliva | 720NHA | Bicine | N.D. |
| 3-36 | saliva | 720NHA | 1,1,1-Tris(hydroxymethyl)ethane | N.D. |
| 3-37 | saliva | 720NHA | Tetrahydropyran | 22.3 |
| 3-38 | saliva | 720NHA | Tetrahydrofuran | 24.7 |
| 3-39 | saliva | 720NHA | 1,3-Dioxolane | 22.5 |
| 3-40 | saliva | 720NHA | 1,4-Dioxane | N.D. |
| 3-41 | saliva | no | no | N.D. |
| 3-42 | saliva | no | Tris | N.D. |

When the nucleic acid extraction reagent containing no test compound was used (sample 3-3), the detection time was 22.1 minutes. This suggests that the test compounds contained in samples with a detection time shorter than 22.1 minutes (sample 3-3) improved the effect of the zeolite adsorbing substances other than nucleic acids. Such compounds were compounds contained in the samples 3-4 to 3-13, 3-15 to 3-21, and 3-23 to 3-29. All of these compounds were proton acceptors and were compounds represented by General Formula (1). On the other hand, the compounds contained in the samples 3-14, 3-22, and 3-30 to 3-40 did not improve the above effect. These compounds were neither proton acceptors nor compounds represented by General Formula (1).

tert-Butylamine has a partial structure of Tris excluding its three hydroxyl groups. In addition, 1,1,1-tris(hydroxymethyl)ethane has a partial structure of Tris excluding its amino group. In the samples containing tert-butylamine and 1,1,1-tris(hydroxymethyl)ethane (samples 3-5 and 3-36), in the sample 3-5, the effect of the zeolite adsorbing substances other than nucleic acids was improved, but in the sample 3-36, the effect was not improved. Accordingly, it was thought that the amino group of Tris was related to the reason why the sample containing Tris improved the above effect. The amino group is a functional group containing a nitrogen atom, and since the nitrogen atom has a lone pair of electrons, a proton forms a coordinate bond with it. In view of such characteristics, it is conceivable that one possible reason for the improvement in the above effect is that Tris functions as a proton acceptor.

In fact, the compound that improved the above effect was a proton acceptor. In addition, aniline (refer to sample 3-14), which is a compound that did not improve the above effect, has a nitrogen atom and an aromatic hydrocarbon group bonded to the carbon atom closest to the nitrogen atom, and thus is not a compound represented by General Formula (1). In addition, pyrrole (refer to sample 3-22) has a nitrogen atom and forms a cyclic structure with a double bond, but does not contain a nitrogen atom other than the nitrogen atom, and thus is not a compound represented by General Formula (1). Aniline and pyrrole cannot be regarded as proton acceptors because the lone pair of electrons on the nitrogen atom were delocalized due to the resonance effect, making it difficult for protons to form a coordinate bond. In addition, cysteine, glycine, proline, and serine (refer to samples 3-30 to 3-33), which are compounds that did not improve the above effect, have a greater number of moles of carboxylic acid groups that donated protons than the number of moles of nitrogen atoms that accepted protons, and thus are not compounds represented by General Formula (1), and cannot be regarded as proton acceptors. Accordingly, it is conceivable that whether the compound is a proton acceptor is important in determining whether the effect of the zeolite adsorbing substances other than nucleic acids is improved.

### [Test Example 4: Confirmation of RNA adsorption to various zeolites]

In order to remove substances inhibiting the nucleic acid amplification reaction (hereinafter referred to as a nucleic acid amplification inhibiting substance), which were contained in the nucleic acid extraction solution prepared from a biological specimen, using the zeolite, it is a prerequisite that the target nucleic acid to be detected not adsorb to the zeolite. Therefore, it was tested whether the target nucleic acid adsorbed to the zeolite.

480 mg of the zeolite described below was added to 1,000 µL of a solution in which SARS-CoV-2 transcription RNA was added to a nucleic acid extraction reagent (0.5% SDS, 50 mM Tris-HCl (pH 7.0)), and the mixture was mixed by inversion. The zeolites used are zeolites commercially available from Tosoh Corporation: model numbers 930NHA (beta-NH₄ type), 940NHA (beta-NH₄ type), 840NHA (ZSM-5-NH₄ type), 720NHA (ferrierite-NH₄ type), 720KOA (ferrierite-K type), and 320NAA (Y-Na type). The suspension mixed by inversion was passed through a 0.45 µm filter, the zeolite and substances adsorbed thereon were removed, and the obtained filtrates were used as zeolite-treated samples 4-1 to 4-6. The target nucleic acid was amplified using 5 µL of the zeolite-treated sample and 20 µL of the quantitative PCR reaction reagent according to a quantitative PCR method. In addition, as a control, a sample 4-7 in which SARS-CoV-2 transcription RNA was added to a nucleic acid extraction reagent but no zeolite was added was also prepared, and subjected to target nucleic acid amplification according to the quantitative PCR method in the same manner.

Amplification of the target nucleic acid according to the quantitative PCR method was performed using the primer set shown in Table 5, according to the reaction under the temperature and time shown in Table 7 in the quantitative PCR reaction reagent solution having the composition shown in Table 6, and the fluorescence value, which increases according target nucleic acid amplification was measured in real time using a Thermal Cycler Dice (registered trademark) Real Time System III. Here, in the probe shown in Table 5, the base sequence shown in SEQ ID NO 3 was labeled with FAM at the 5' end and labeled with BHQ1 at the 3' end. Table 8 shows the results of confirmation of amplification according to the quantitative PCR method. Here, in Table 8, the "proportion of copies" is the percentage relative to the number of copies of the "no zeolite treatment" sample (defined as 100%).

**[Table 5]**

| <Primer and probe set for quantitative PCR method targeting SARS-CoV-2 RNA as nucleic acid> | |
|---|---|
| Name | Sequence (5'→3') |
| Forward primer | AAATTTTGGGGACCAGGAAC (SEQ ID NO 1) |
| Reverse primer | TGGCAGCTGTGTAGGTCAAC (SEQ ID NO 2) |
| Probe | FAM-ATGTCGCGCATTGGCATGGA (SEQ ID NO 3)-BHQ1 |

**[Table 6]**

| <Composition of quantitative PCR reaction reagent (20 µL)> |
|---|
| 1x One Step PrimeScript III RT-qPCR Mix |
| 0.4 µM forward primer |
| 0.4 µM reverse primer |
| 0.2 µM probe |
| Distilled water |

**[Table 7]**

| <Reaction conditions> | | |
|---|---|---|
| Step 1 | 52°C 5 min, 95°C 10 sec | 1 cycle |
| Step 2 | 95°C 5 sec→60°C 30 sec | 40 cycles |

**[Table 8]**

| Sample No. | Zeolite treatment | | | Number of copies (copies/test) | Proportion of copies |
|---|---|---|---|---|---|
| | Model number | Crystal structure | Cation | | |
| 4-1 | 930NHA | beta | NH₄ | 3,529 | 93% |
| 4-2 | 940NHA | beta | NH₄ | 3,282 | 87% |
| 4-3 | 840NHA | ZSM-5 | NH₄ | 3,454 | 91% |
| 4-4 | 720NHA | ferrierite | NH₄ | 3,422 | 90% |
| 4-5 | 720KOA | ferrierite | K | 3,501 | 92% |
| 4-6 | 320NAA | Y type | Na | 3,678 | 97% |
| 4-7 | no zeolite treatment | | | 3,789 | 100% |

In the zeolite-treated samples 4-1 to 4-6, target nucleic acid amplification was confirmed, and the degree of amplification (number of copies) was comparable to that of the control sample 4-7. This suggests that almost no RNA was adsorbed to the zeolite in the presence of Tris-HCl.

### [Test Example 5: Confirmation of DNA adsorption to various zeolites]

Zeolite-treated samples 5-1 to 5-6 were obtained in the same manner as in Test Example 4 except that DNA of Saccharomyces cerevisiae (hereinafter referred to as "S. cerevisiae") was added in place of SARS-CoV-2 transcription RNA. In addition, as a control, a sample 5-7 in which S. cerevisiae DNA was added to a nucleic acid extraction reagent, but no zeolite was added was also prepared. These samples were subjected to target nucleic acid (*S. cerevisiae* DNA) amplification according to the quantitative PCR method in the same manner as in Test Example 4 except that the reaction was performed using the primer and probe set shown in Table 9, and the quantitative PCR reaction reagent solution shown in Table 10 under the temperature and time shown in Table 11, and the fluorescence value, which increases according target nucleic acid amplification was measured in real time. Here, in the probe shown in Table 9, the base sequence shown in SEQ ID NO 6 was labeled with FAM at the 5' end and labeled with BHQ1 at the 3' end. Table 12 shows the results of confirmation of amplification according to the quantitative PCR method. Here, in Table 12, the "proportion of copies" is the percentage relative to the number of copies of the "no zeolite treatment" sample (defined as 100%).

**[Table 9]**

| <Primer and probe set for quantitative PCR method targeting *S. cerevisiae* DNA as nucleic acid> | |
|---|---|
| Name | Sequence (5'→3') |
| Forward primer | GGACTCTGGACATGCAAGAT (SEQ ID NO 4) |
| Reverse primer | ATACCCTTCTTAACACCTGGC (SEQ ID NO 5) |
| Probe | FAM-CCCTTCAGAGCGTTTTCTCTAAATTGATAC (SEQ ID NO 6)-BHQ1 |

**[Table 10]**

| <Composition of quantitative PCR reaction reagent (20 µL)> |
|---|
| 1x Premix Ex Taq (Probe qPCR) |
| 0.5 µM forward primer |
| 0.5 µM reverse primer |
| 0.2 µM probe |
| Distilled water |

**[Table 11]**

| <Reaction conditions> | | |
|---|---|---|
| Step 1 | 95°C 30 sec | 1 cycle |
| Step 2 | 95°C 5 sec→60°C 30 sec | 40 cycles |

**[Table 12]**

| Sample No. | Zeolite treatment | | | Number of copies (copies/test) | Proportion of copies |
|---|---|---|---|---|---|
| | Model number | Crystal structure | Cation | | |
| 5-1 | 930NHA | beta | NH₄ | 2,188 | 82% |
| 5-2 | 940NHA | beta | NH₄ | 2,098 | 79% |
| 5-3 | 840NHA | ZSM-5 | NH₄ | 2,788 | 105% |
| 5-4 | 720NHA | ferrierite | NH₄ | 2,450 | 92% |
| 5-5 | 720KOA | ferrierite | K | 2,534 | 95% |
| 5-6 | 320NAA | Y type | Na | 2,988 | 113% |
| 5-7 | no zeolite treatment | | | 2,656 | 100% |

Similar results were obtained with DNA as with RNA. In the zeolite-treated samples 5-1 to 5-6, target nucleic acid amplification was confirmed, and the degree of amplification (number of copies) was comparable to that of the control sample 5-7. This suggests that almost no DNA was adsorbed to the zeolite in the presence of Tris-HCl.

### [Test Example 6: Evaluation of RNA extraction and purification using various zeolites]

It was examined whether the improved adsorption effect by Tris-HCl confirmed with the zeolite 720NHA could also be obtained with other types of zeolites.

Zeolite-treated samples 6-5 to 6-16 were obtained in the same manner as in Test Example 1 except that, in addition to 720NHA, model number 690HOA, 642NAA, 840NHA, 722HOA, or 720KOA (commercially available from Tosoh Corporation) was used as the zeolite. In addition, as positive controls, samples 6-1 and 6-2 obtained by adding distilled water (DW) in place of the saliva specimen and performing mixing were prepared. In addition, as negative controls, zeolite-untreated samples 6-3 and 6-4 obtained by mixing without adding the zeolite were also prepared. In the same manner as in Test Example 1, the obtained samples were subjected to target nucleic acid (SARS-CoV-2 transcription RNA) amplification using a Loopamp (registered trademark) Novel Coronavirus 2019 (SARS-CoV-2) detection reagent kit (commercially available from Eiken Chemical Co., Ltd.), and the turbidity value, which increases according to amplification of the target nucleic acid, was measured in real time. The results are shown in Table 13. In Table 13, samples in which no detection occurred within the measurement time (90 minutes) are indicated as "N.D.".

**[Table 13]**

| Sample No. | Specimen | Zeolite treatment | | | Presence of Tris-HCl | Detection time (min) |
|---|---|---|---|---|---|---|
| | | Model number | Crystal structure | Cation | | |
| 6-1 | DW | no zeolite treatment | | | no | 15.6 |
| 6-2 | DW | no zeolite treatment | | | yes | 16.9 |
| 6-3 | saliva | no zeolite treatment | | | no | N.D. |
| 6-4 | saliva | no zeolite treatment | | | yes | N.D. |
| 6-5 | saliva | 690HOA | mordenite | H | no | 28.4 |
| 6-6 | saliva | 690HOA | mordenite | H | yes | 20.0 |
| 6-7 | saliva | 642NAA | mordenite | Na | no | 43.9 |
| 6-8 | saliva | 642NAA | mordenite | Na | yes | 26.7 |
| 6-9 | saliva | 840NHA | ZSM-5 | NH₄ | no | 61.3 |
| 6-10 | saliva | 840NHA | ZSM-5 | NH₄ | yes | 40.5 |
| 6-11 | saliva | 722HOA | ferrierite | H | no | 43.9 |
| 6-12 | saliva | 722HOA | ferrierite | H | yes | 19.2 |
| 6-13 | saliva | 720NHA | ferrierite | NH₄ | no | 51.7 |
| 6-14 | saliva | 720NHA | ferrierite | NH₄ | yes | 19.3 |
| 6-15 | saliva | 720KOA | ferrierite | K | no | 31.3 |
| 6-16 | saliva | 720KOA | ferrierite | K | yes | 19.1 |

In the samples containing no saliva, even without the zeolite treatment, target nucleic acid amplification was confirmed (samples 6-1 and 6-2). In addition, nucleic acid amplification was hardly affected by the presence or absence of the buffer. On the other hand, in the zeolite-untreated samples containing a saliva specimen, regardless of the presence or absence of the buffer, target nucleic acid amplification was not confirmed (samples 6-3 and 6-4). In addition, in all of the zeolite-treated samples containing a saliva specimen (6-5 to 6-16), the detection time for the target nucleic acid was significantly shorter when Tris-HCl was present than when Tris-HCl was not present. These results suggest that, when the sample containing nucleic acids was brought into contact with the zeolite and Tris-HCl, it was possible to improve the effect of the zeolite adsorbing substances other than nucleic acids, and to extract and purify nucleic acids easily, rapidly, and with high purity.

### [Test Example 7: Evaluation of extraction and purification of DNA using various zeolites]

Zeolite-treated samples 7-5 to 7-8 were obtained in the same manner as in Test Example 6 except that DNA of Bordetella pertussis (hereinafter referred to as "B. pertussis") was used in place of SARS-CoV-2 transcription RNA as the target nucleic acid, and 720NHA or 840NHA was used as the zeolite. In addition, as positive controls, samples 7-1 and 7-2 in which distilled water (DW) was added in place of the saliva specimen and mixed were prepared. In addition, as negative controls, zeolite-untreated samples 7-3 and 7-4 obtained by mixing without adding the zeolite were also prepared. To 11.5 µL of the obtained sample , 11.5 µL of distilled water and 2 µL of the target nucleic acid solution (2,000 copies/test) were added, the mixture was reacted using a Simprova (registered trademark) Respiratory Infection Panel (commercially available from Eiken Chemical Co., Ltd.) at 64°C for 50 minutes, the target nucleic acid was amplified, and the fluorescence value, which increases as a result of target nucleic acid amplification, was measured using a Thermal Cycler Dice (registered trademark) Real Time System III. The results are shown in Table 14. In Table 14, samples in which no detection occurred within the measurement time (50 minutes) are indicated as "N. D.".

**[Table 14]**

| Sample No. | Specimen | Zeolite treatment | | | Presence of Tris-HCl | Detection time (min) |
|---|---|---|---|---|---|---|
| | | Model number | Crystal structure | Cation | | |
| 7-1 | DW | no zeolite treatment | | | no | 8.1 |
| 7-2 | DW | no zeolite treatment | | | yes | 7.8 |
| 7-3 | saliva | no zeolite treatment | | | no | N.D. |
| 7-4 | saliva | no zeolite treatment | | | yes | N.D. |
| 7-5 | saliva | 840NHA | ZSM-5 | NH₄ | no | 13.9 |
| 7-6 | saliva | 840NHA | ZSM-5 | NH₄ | yes | 9.9 |
| 7-7 | saliva | 720NHA | ferrierite | NH₄ | no | 13.9 |
| 7-8 | saliva | 720NHA | ferrierite | NH₄ | yes | 9.2 |

In the samples containing no saliva, even without the zeolite treatment, target nucleic acid amplification was confirmed (samples 7-1 and 7-2). In addition, nucleic acid amplification was hardly affected by the presence or absence of the buffer. On the other hand, in the zeolite-untreated samples containing a saliva specimen, regardless of the presence or absence of the buffer, target nucleic acid amplification was not confirmed (samples 7-3 and 7-4). In addition, in all of the zeolite-treated samples containing a saliva specimen (7-5 to 7-8), the detection time for the target nucleic acid was significantly shorter when Tris-HCl was present than when Tris-HCl was not present. These results suggest that, when the sample containing nucleic acids was brought into contact with the zeolite and Tris-HCl, it was possible to improve the effect of the zeolite adsorbing substances other than nucleic acids and to extract and purify nucleic acids easily, rapidly, and with high purity.

### [Test Example 8: Application of RNA purified using zeolite to PCR]

200 µL of a solution obtained by adding influenza A virus to a saliva specimen and a nucleic acid extraction reagent containing 0.5% SDS or a nucleic acid extraction reagent containing 0.5% SDS+50 mM Tris-HCl (pH 7.0) were mixed. Then, 240 mg of a zeolite (model number 720NHA, commercially available from Tosoh Corporation) was added, and the mixture was mixed by inversion. The suspension mixed by inversion was passed through a 0.45 µm filter to obtain zeolite-treated samples 8-6 and 8-8. In addition, as positive controls, samples 8-1 to 8-4 obtained by adding and mixing saline in place of the saliva specimen were prepared. In addition, as negative controls, zeolite-untreated samples 8-5 and 8-7 obtained by mixing without adding the zeolite were also prepared. The target nucleic acid was amplified using 5 µL of each of the obtained samples and 20 µL of the quantitative PCR reaction reagent according to the quantitative PCR method.

Amplification of the target nucleic acid according to the quantitative PCR method was performed using the primer set shown in Table 15, according to the reaction under the temperature and time shown in Table 7 in the quantitative PCR reaction reagent solution shown in Table 6, and the fluorescence value, which increases as a result of target nucleic acid amplification, was measured in real time using a LightCycler (registered trademark) 480 System. Here, the probe composed of a base sequence shown in SEQ ID NO 9 in Table 15 was labeled with FAM at the 5' end and labeled with MGB at the 3' end. The results are shown in Table 16. Here, in Table 16, the "proportion of copies" is the percentage relative to the number of copies of the sample 8-1 (defined as 100%).

**[Table 15]**

| <Primer and probe set for quantitative PCR method targeting influenza A virus RNA as nucleic acid> | |
|---|---|
| Name | Sequence (5'→3') |
| Forward primer | CCMAGGTCGAAACGTAYGTTCTCTCTATC (SEQ ID NO 7) |
| Reverse primer | TGACAGRATYGGTCTTGTCTTTAGCCAYTCCA (SEQ ID NO 8) |
| Probe | FAM-CAGCCAGCAATRTTRCATTTACC (SEQ ID NO 9)-MGB |

**[Table 16]**

| Sample No. | Specimen | Presence of Tris-HCl | Zeolite treatment | Number of copies (copies/test) | Proportion of copies |
|---|---|---|---|---|---|
| 8-1 | virus+saline | no | no | 282,662 | 100% |
| 8-2 | virus+saline | no | 720NHA | 311,318 | 110% |
| 8-3 | virus+saline | ves | no | 295,203 | 104% |
| 8-4 | virus+saline | yes | 720NHA | 322,933 | 114% |
| 8-5 | virus+saliva | no | no | 149,132 | 53% |
| 8-6 | virus+saliva | no | 720NHA | 187,030 | 66% |
| 8-7 | virus+saliva | yes | no | 114,252 | 40% |
| 8-8 | virus+saliva | yes | 720NHA | 268,013 | 95% |

In the samples containing saline in place of the saliva specimen (samples 8-1 to 8-4), regardless of the presence or absence of zeolite treatment or Tris-HCl, target nucleic acid amplification was confirmed. Regardless of the presence or absence of Tris-HCl, the negative control zeolite-untreated samples (samples 8-5 and 8-7) exhibited amplification that was only about half that of the positive control. These results suggest that nucleic acid amplification was inhibited by the saliva specimen. In addition, in the sample containing only influenza A virus and a saliva specimen (sample 8-6), the target nucleic acid was detected at only about half the number of copies observed in the samples containing saline in place of the saliva specimen (samples 8-1 to 8-4). That is, in the sample 8-6, the target nucleic acid was detected at only about a number of copies comparable to that of the negative control (sample 8-5). On the other hand, in the sample containing influenza A virus, a saliva specimen and Tris-HCl (sample 8-8), the target nucleic acid was detected at almost the same number of copies as that of the samples containing saline in place of the saliva specimen (samples 8-1 to 8-4). These results suggest that, when the sample containing nucleic acids was brought into contact with the zeolite and Tris-HCl, it was possible to improve the effect of the zeolite adsorbing substances other than nucleic acids and to extract and purify nucleic acids easily, rapidly, and with high purity.

### [Test Example 9: Application of DNA purified using zeolite to PCR]

200 µL of a solution obtained by adding Streptococcus pneumoniae (hereinafter referred to as "S. pneumoniae") to a saliva specimen, and a nucleic acid extraction reagent containing 0.5% SDS, or a nucleic acid extraction reagent containing 0.5% SDS+50 mM Tris-HCl (pH 7.0) were mixed, 240 mg of a zeolite (model number 720NHA, commercially available from Tosoh Corporation) was added, and the mixture was mixed by inversion. The suspension mixed by inversion was passed through a 0.45 µm filter to obtain zeolite-treated samples 9-6 and 9-8. In addition, as positive controls, samples 9-1 to 9-4 obtained by adding saline in place of the saliva specimen and performing mixing were prepared. In addition, as negative controls, zeolite-untreated samples 9-5 and 9-7 obtained by mixing without adding the zeolite were also prepared. The target nucleic acid was amplified using 5 µL of each of the obtained samples and 20 µL of the quantitative PCR reaction reagent according to the quantitative PCR method.

Amplification of the target nucleic acid according to the quantitative PCR method was performed using the primer and probe set shown in Table 17, according to the reaction under the temperature and time shown in Table 19 in the quantitative PCR reaction reagent solution having the composition shown in Table 18, and the fluorescence value, which increases according target nucleic acid amplification was measured in real time using a LightCycler (registered trademark) 480 System. Here, in the probe shown in Table 17, the base sequence shown in SEQ ID NO 12 was labeled with FAM at the 5' end and labeled with TAMRA at the 3' end. Table 20 shows the results of confirmation of amplification according to the quantitative PCR method. Here, in Table 20, the "proportion of copies" is the percentage relative to the number of copies of the sample 9-1 (defined as 100%).

**[Table 17]**

| <Primer and probe set for quantitative PCR method targeting *S. pneumoniae* DNA as nucleic acid> | |
|---|---|
| Name | Sequence (5'→3') |
| Forward primer | ACGCAATCTAGCAGATGAAGC (SEQ ID NO 10) |
| Reverse primer | TGTTTGGTTGGTTATTCGTGC (SEQ ID NO 11) |
| Probe | FAM-TTTGCCGAAAACGCTTGATACAGGG (SEQ ID NO 12)-TAMRA |

**[Table 18]**

| <Composition of quantitative PCR reaction reagent (20 µL)> |
|---|
| 1× Premix Ex Taq (Probe qPCR) |
| 0.2 µM forward primer |
| 0.2 µM reverse primer |
| 0.2 µM probe |
| Distilled water |

**[Table 19]**

| <Reaction conditions> | | |
|---|---|---|
| Step 1 | 95°C 30 sec | 1 cycle |
| Step 2 | 95°C 5 sec→60°C 30 sec | 40 cycles |

**[Table 20]**

| Sample No. | Specimen | Presence of Tris-HCl | Zeolite treatment | Number of copies (copies/test) | Proportion of copies |
|---|---|---|---|---|---|
| 9-1 | bacteria+saline | no | no | 106,423 | 100% |
| 9-2 | bacteria+saline | no | yes | 106,012 | 100% |
| 9-3 | bacteria+saline | yes | no | 108,516 | 102% |
| 9-4 | bacteria+saline | yes | yes | 104,390 | 98% |
| 9-5 | bacteria+saliva | no | no | 74,415 | 70% |
| 9-6 | bacteria+saliva | no | yes | 92,129 | 87% |
| 9-7 | bacteria+saliva | yes | no | 77,396 | 73% |
| 9-8 | bacteria+saliva | yes | yes | 113,916 | 107% |

In the samples containing saline in place of the saliva specimen (samples 9-1 to 9-4), regardless of the presence or absence of zeolite treatment or Tris-HCl, target nucleic acid amplification was confirmed. Regardless of the presence or absence of Tris-HCl, the negative control zeolite-untreated samples (samples 9-5 and 9-7) exhibited a lower amplification rate (smaller number of copies) than that of the positive control. These results suggest that nucleic acid amplification was inhibited by the saliva specimen. In addition, in the sample (sample 9-6) containing only S. Pneumoniae and a saliva specimen in a nucleic acid extraction reagent, the target nucleic acid was detected at a lower number of copies than in the positive control samples containing saline in place of the saliva specimen (samples 9-1 to 9-4). On the other hand, in the sample (sample 9-8) containing S. Pneumoniae, a saliva specimen and Tris-HCl, the target nucleic acid was detected at almost the same number of copies as that of the samples containing saline in place of the saliva specimen (samples 9-1 to 9-4). These results suggest that, when the sample containing nucleic acids was brought into contact with the zeolite and Tris-HCl, it was possible to improve the effect of the zeolite adsorbing substances other than nucleic acids and to extract and purify nucleic acids easily, rapidly, and with high purity.

### [Test Example 10: Application of RNA purified using zeolite to PCR in other specimens]

Zeolite-treated samples were obtained in the same manner as in Test Example 1 except that a mordenite-H-type zeolite or a ferrierite-NH₄-type zeolite (model number: 690HOA or 720NHA, commercially available from Tosoh Corporation) was used as the zeolite, and a nucleic acid extraction reagent was mixed with various specimens (serum, urine, sputum, nasal swab, cerebrospinal fluid, feces, whole blood). In addition, as a positive control, a sample in which distilled water (DW) was added in place of the saliva specimen and mixed was prepared. In addition, as a negative control, a zeolite-untreated sample which was mixed without adding the zeolite was also prepared. In the same manner as in Test Example 1, the obtained samples were subjected to target nucleic acid (SARS-CoV-2 transcription RNA) amplification (250 copies/test) using a Loopamp (registered trademark) Novel Coronavirus 2019 (SARS-CoV-2) detection reagent kit (commercially available from Eiken Chemical Co., Ltd.), and the turbidity value, which increases according to amplification of the target nucleic acid, was measured in real time. The results are shown in Tables 21 to 26. In Tables 21 to 26, samples in which no detection occurred within the measurement time (35 minutes) are indicated as "N. D.".

**[Table 21]**

| Sample No. | Specimen | Presence of Tris-HCl | Zeolite treatment | Detection time (min) |
|---|---|---|---|---|
| 10-1 | DW | no | no | 15.3 |
| 10-2 | serum | no | no | N.D. |
| 10-3 | serum | no | 720NHA | N.D. |
| 10-4 | serum | no | 690HOA | N.D. |
| 10-5 | serum | yes | no | N.D. |
| 10-6 | serum | yes | 720NHA | 21.8 |
| 10-7 | serum | yes | 690HOA | 24.0 |
| 10-8 | urine | no | no | N.D. |
| 10-9 | urine | no | 720NHA | N.D. |
| 10-10 | urine | no | 690HOA | N.D. |
| 10-11 | urine | yes | no | N.D. |
| 10-12 | urine | yes | 720NHA | 22.8 |
| 10-13 | urine | yes | 690HOA | 27.0 |

**[Table 22]**

| Sample No. | Specimen | Presence of Tris-HCl | Zeolite treatment | Detection time (min) |
|---|---|---|---|---|
| 10-1 | DW | no | no | 15.7 |
| 10-14 | sputum | no | no | N.D. |
| 10-15 | sputum | no | 720NHA | 26.5 |
| 10-16 | sputum | no | 690HOA | N.D. |
| 10-17 | sputum | yes | no | N.D. |
| 10-18 | sputum | yes | 720NHA | 18.6 |
| 10-19 | sputum | yes | 690HOA | 32.5 |

**[Table 23]**

| Sample No. | Specimen | Presence of Tris-HCl | Zeolite treatment | Detection time (min) |
|---|---|---|---|---|
| 10-1 | DW | no | no | 15.7 |
| 10-20 | swab | no | no | N.D. |
| 10-21 | swab | no | 720NHA | N.D. |
| 10-22 | swab | yes | no | N.D. |
| 10-23 | swab | yes | 720NHA | 24.4 |

**[Table 24]**

| Sample No. | Specimen | Presence of Tris-HCl | Zeolite treatment | Detection time (min) |
|---|---|---|---|---|
| 10-1 | DW | no | no | 16.0 |
| 10-24 | cerebrospinal fluid | no | no | N.D. |
| 10-25 | cerebrospinal fluid | no | 720NHA | 24.6 |
| 10-26 | cerebrospinal fluid | yes | no | N.D. |
| 10-27 | cerebrospinal fluid | yes | 720NHA | 20.9 |

**[Table 25]**

| Sample No. | Specimen | Presence of Tris-HCl | Zeolite treatment | Detection time (min) |
|---|---|---|---|---|
| 10-1 | DW | no | no | 15.8 |
| 10-28 | feces | no | no | N.D. |
| 10-29 | feces | no | 720NHA | N.D. |
| 10-30 | feces | no | 690HOA | N.D. |
| 10-31 | feces | yes | no | N.D. |
| 10-32 | feces | yes | 720NHA | 26.3 |
| 10-33 | feces | yes | 690HOA | 20.5 |

**[Table 26]**

| Sample No. | Specimen | Presence of Tris-HCl | Zeolite treatment | Detection time (min) |
|---|---|---|---|---|
| 10-1 | DW | no | no | 15.8 |
| 10-34 | whole blood | no | no | N.D. |
| 10-35 | whole blood | no | 720NHA | N.D. |
| 10-36 | whole blood | no | 690HOA | N.D. |
| 10-37 | whole blood | yes | no | 24.2 |
| 10-38 | whole blood | yes | 720NHA | 19.0 |
| 10-39 | whole blood | yes | 690HOA | 17.7 |

Regardless of which specimen was used, the detection time for the target nucleic acid was shortened in the zeolite-treated sample containing Tris-HCl.

## Claims

1. A nucleic acid purification method, comprising a step of bringing a sample containing a nucleic acid into contact with a zeolite and a proton acceptor.

2. The method according to claim 1, wherein the proton acceptor is a compound represented by General Formula (1): 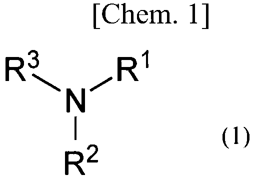 (in the formula, R¹, R² and R³ are each independently a hydrogen atom, an optionally substituted aliphatic hydrocarbon group having 1 to 6 carbon atoms, or an optionally substituted chain aliphatic hydrocarbon group having 2 to 10 carbon atoms and in which a nitrogen atom, a sulfur atom or an oxygen atom is interposed between carbon atoms, the substituent is selected from the group consisting of a hydroxyl group, a carboxyl group, an amino group, a sulfonic acid group, an imino group, a guanidyl group, an aromatic hydrocarbon group having 6 to 12 carbon atoms, and a 3- to 6-membered heterocyclic group which contains, as a ring-constituting atom, at least one atom selected from the group consisting of a nitrogen atom, a sulfur atom and an oxygen atom, and which may have a substituent that is an aliphatic hydrocarbon group having 1 to 6 carbon atoms,
when the optionally substituted aliphatic hydrocarbon group having 1 to 6 carbon atoms has a substituent that is an aromatic hydrocarbon group having 6 to 12 carbon atoms, the aliphatic hydrocarbon group having 1 to 6 carbon atoms is an aliphatic hydrocarbon group having 2 to 6 carbon atoms, and the aromatic hydrocarbon group is not bonded to the carbon atom of the aliphatic hydrocarbon group having 2 to 6 carbon atoms that is closest to the N atom in General Formula (1),
R¹ and R², R² and R³, or R¹ and R³ may be bonded to form a cyclic structure, and in this case, the cyclic structure is a 5- to 7-membered ring, and may further include a nitrogen atom, a sulfur atom or an oxygen atom, and when the cyclic structure has a double bond, it contains a nitrogen atom other than the N atom in General Formula (1), and
in General Formula (1), the total number of primary, secondary or tertiary amino groups is greater than the total number of -COOH and -SO₃H groups).

3. The method according to claim 1, wherein the proton acceptor is tris(hydroxymethyl)aminomethane and/or 2-morpholinoethanesulfonic acid.

4. The method according to any one of claims 1 to 3, wherein the sample is derived from at least one specimen selected from the group consisting of blood, cerebrospinal fluid, urine, feces, sputum, saliva, nasal discharge, swab fluid, amniotic fluid, and gargle fluid.

5. The method according to any one of claims 1 to 3, wherein, in the contacting step, at least a portion of the nucleic acid is not adsorbed to the zeolite.

6. The method according to any one of claims 1 to 3, wherein the zeolite is at least one selected from the group consisting of a ferrierite-type zeolite, a ZSM-5-type zeolite, a Y-type zeolite, a beta-type zeolite, and a mordenite type.

7. The method according to any one of claims 1 to 3, wherein the zeolite is at least one selected from the group consisting of a ferrierite-NH₄-type zeolite, a ferrierite-K-type zeolite, a ZSM-5-NH₄-type zeolite, a Y-Na-type zeolite, a beta-NH₄-type zeolite, a mordenite-H-type zeolite, a mordenite-Na type, and a ferrierite-H-type zeolite.

8. The method according to any one of claims 1 to 3, wherein the nucleic acid is RNA and/or DNA.

9. A kit for purifying a nucleic acid, comprising a zeolite and a proton acceptor.

10. The kit according to claim 9, wherein the proton acceptor is a compound represented by General Formula (1): 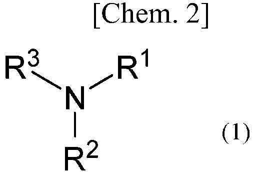 (in the formula, R¹, R² and R³ are each independently a hydrogen atom, an optionally substituted aliphatic hydrocarbon group having 1 to 6 carbon atoms, or an optionally substituted chain aliphatic hydrocarbon group having 2 to 10 carbon atoms and in which a nitrogen atom, a sulfur atom or an oxygen atom is interposed between carbon atoms, the substituent is selected from the group consisting of a hydroxyl group, a carboxyl group, an amino group, a sulfonic acid group, an imino group, a guanidyl group, an aromatic hydrocarbon group having 6 to 12 carbon atoms, and a 3- to 6-membered heterocyclic group which contains, as a ring-constituting atom, at least one atom selected from the group consisting of a nitrogen atom, a sulfur atom and an oxygen atom, and which may have a substituent that is an aliphatic hydrocarbon group having 1 to 6 carbon atoms,
when the optionally substituted aliphatic hydrocarbon group having 1 to 6 carbon atoms has a substituent that is an aromatic hydrocarbon group having 6 to 12 carbon atoms, the aliphatic hydrocarbon group having 1 to 6 carbon atoms is an aliphatic hydrocarbon group having 2 to 6 carbon atoms, and the aromatic hydrocarbon group is not bonded to the carbon atom of the aliphatic hydrocarbon group having 2 to 6 carbon atoms that is closest to the N atom in General Formula (1),
R¹ and R², R² and R³, or R¹ and R³ may be bonded to form a cyclic structure, and in this case, the cyclic structure is a 5- to 7-membered ring, and may further include a nitrogen atom, a sulfur atom or an oxygen atom, and when the cyclic structure has a double bond, it contains a nitrogen atom other than the N atom in General Formula (1), and
in General Formula (1), the total number of primary, secondary or tertiary amino groups is greater than the total number of -COOH and -SO₃H groups).

11. The kit according to claim 9, wherein the proton acceptor is tris(hydroxymethyl)aminomethane and/or 2-morpholinoethanesulfonic acid.

12. The kit according to any one of claims 9 to 11, wherein the zeolite is at least one selected from the group consisting of a ferrierite-type zeolite, a ZSM-5-type zeolite, a Y-type zeolite, a beta-type zeolite, and a mordenite type.

13. The kit according to any one of claims 9 to 11, wherein the zeolite is at least one selected from the group consisting of a ferrierite-NH₄-type zeolite, a ferrierite-K-type zeolite, a ZSM-5-NH₄-type zeolite, a Y-Na-type zeolite, a beta-NH₄-type zeolite, a mordenite-H-type zeolite, a mordenite-Na type, and a ferrierite-H-type zeolite.

14. The kit according to any one of claims 9 to 11, wherein the kit is a kit for purifying a nucleic acid derived from at least one specimen selected from the group consisting of blood, cerebrospinal fluid, urine, feces, sputum, saliva, nasal discharge, swab fluid, amniotic fluid, and gargle fluid, and further includes a nucleic acid extraction reagent containing a surfactant.

15. The kit according to any one of claims 9 to 11, wherein the nucleic acid is RNA and/or DNA.
